(19) Europäisches Patentamt
European Patent Office
Office européen des brevets

(11) **EP 3 821 794 B1**

(12) **EUROPEAN PATENT SPECIFICATION**

(45) Date of publication and mention
of the grant of the patent:
**15.02.2023   Bulletin 2023/07**

(21) Application number: **20215654.3**

(22) Date of filing: **14.12.2017**

(51) International Patent Classification (IPC):
**A61B 5/00** *(1968.09)*      **A61B 18/14** *(2000.01)*
**A61B 18/00** *(2000.01)*      **A61B 17/00** *(1968.09)*
**A61B 34/20** *(2016.01)*      **A61B 90/00** *(2016.01)*

(52) Cooperative Patent Classification (CPC):
**A61B 18/1492; A61B 5/6852; A61B 5/6885;**
A61B 2017/00221; A61B 2018/00351;
A61B 2018/00357; A61B 2018/00577;
A61B 2034/2051; A61B 2090/064; A61B 2090/065;
A61B 2090/376; A61B 2562/0252; A61B 2562/0261

(54) **WIRELESS FORCE SENSOR**

DRAHTLOSER KRAFTSENSOR

CAPTEUR DE FORCE SANS FIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **16.12.2016   US 201662435287 P**

(43) Date of publication of application:
**19.05.2021   Bulletin 2021/20**

(62) Document number(s) of the earlier application(s) in
accordance with Art. 76 EPC:
**17835706.7 / 3 525 664**

(73) Proprietor: **St. Jude Medical International Holding
S.à r.l.
2449 Luxembourg (LU)**

(72) Inventors:
• **ALBU, Ryan**
  **Minneapolis, Minnesota 55419 (US)**
• **EBNER, Bruce**
  **Shorewood, Minnesota 55331 (US)**
• **OLSON, Gregory K.**
  **Elk River, Minnesota 55330 (US)**
• **TEGG, Troy T.**
  **Elk River, Minnesota 55330 (US)**
• **PARK, Jin W.**
  **Suwanee, Georgia 30024 (US)**
• **PARSONAGE, Ed**
  **St. Paul, Minnesota 55116 (US)**

(74) Representative: **Kramer Barske Schmidtchen
Patentanwälte PartG mbB
European Patent Attorneys
Landsberger Strasse 300
80687 München (DE)**

(56) References cited:
EP-A1- 2 338 430      EP-A2- 2 347 726
WO-A1-2017/070322      WO-A2-2007/050960
US-A1- 2010 305 476      US-A1- 2014 276 078
US-B2- 9 101 734

## Description

BACKGROUND

a. Field

The present invention relates to a catheter system for treating cardiac arrhythmias.

b. Background

Within a cardiac cycle, the human heart experiences electrical impulses traversing from the sinus node to the ventricles. Cardiac contraction is driven by a cycle of polarization and depolarization as electrical currents spread across the heart. In healthy hearts, the heart will experience an orderly progression of depolarization waves called sinus rhythm. In unhealthy hearts, such as those experiencing atrial arrhythmia, including for example, ectopic atrial tachycardia, atrial fibrillation, and atrial flutter, the progression of the depolarization wave becomes chaotic. Arrhythmias may persist as a result of scar tissue or other obstacles to rapid and uniform depolarization. These obstacles may cause depolarization waves to electrically circulate through some parts of the heart more than once. Atrial arrhythmia can create a variety of dangerous conditions, including irregular heart rates, loss of synchronous atrioventricular contractions, and blood flow stasis. All of these conditions have been associated with a variety of ailments, including death.

Catheters are used in a variety of diagnostic and/or therapeutic medical procedures to diagnose and correct conditions such as atrial arrhythmia, including for example, ectopic atrial tachycardia, atrial fibrillation, and atrial flutter. Typically, in atrial fibrillation therapies, a catheter is manipulated through a patient's vasculature to the patient's heart carrying one or more electrodes which may be used for mapping, ablation, diagnosis, or other treatment. Where an ablation therapy is desired to alleviate symptoms of atrial fibrillation, the ablation catheter imparts ablative energy to cardiac tissue to create a lesion in the cardiac tissue. The lesioned tissue is less capable of conducting electrical signals, thereby disrupting undesirable electrical pathways and limiting or preventing stray electrical signals that lead to arrhythmias. The ablation catheter may utilize ablative energy including, for example, radio-frequency (RF), cryoablation, laser, chemical, and high-intensity focused ultrasound. Ablation therapies often require precise positioning of the ablation catheter, as well as precise pressure exertion for optimal ablative-energy transfer into the targeted myocardial tissue. Excess force between the ablation catheter tip and the targeted myocardial tissue may result in excessive ablation which may permanently damage the cardiac muscle and/or surrounding nerves. When contact force between the ablation catheter tip and the targeted myocardial tissue is below a target force, the efficacy of the ablation therapy may be reduced, or entirely negated.

Ablation therapies are often delivered by making a number of individual ablations in a controlled fashion in order to form a lesion line. To improve conformity of the individual ablations along the lesion line, it is desirable to precisely control the position at which the individual ablations are conducted, the ablation period, and the contact force between the ablation catheter tip and the targeted tissue. All of these factors affect the conformity of the resulting lesion line. Catheter localization systems, in conjunction with mapping systems, have vastly improved a clinician's ability to precisely position the ablation catheter tip for an ablation and determine the efficacy of a treatment. Similarly, ablation controller circuitry has improved the consistency of individual ablation therapies. There are devices that attempt to measure the force exerted between myocardial tissue and the ablation catheter tip. Existing designs utilize ablation catheter tips with deformable bodies which deform in response to a force being exerted on the ablation catheter tip. Sensors (e.g., magnetic, optical, etc.) are used to approximate the deformation of the deformable body and to output a signal to controller circuitry that associates the deformation with a force exerted by the ablation catheter tip. However, existing deformable body designs suffer from both complexity and cost; related to, for example, acquisition and delivery of the measurement signal to the proximal end of the catheter.

The foregoing discussion is intended only to illustrate the present field and should not be taken as a disavowal of claim scope.

WO 2007/050960 A2 relates to a system for electrode contact assessment comprising the features of the preamble of claim 1.

US 2014/0276078 A1 relates to medical devices including a catheter configured to provide an indication of contact between the catheter and tissue in a body.

EP 2 347 726 A2 relates to an electrophysiologic catheter useful for ablation and sensing electrical activity of heart tissue.

US 9,101,734 B2 relates to a medical device for use in the vessel of a patient for the purpose of diagnosing or treating the patient.

EP 2 338 430 A1 relates to invasive medical devices, in particular to the construction of probes for insertion into body organs.

WO 2017/070322 A1 relates to interventional monitoring, detection, mapping, and diagnostic/therapeutic feedback of autonomic and cardiac electrophysiologic signals and function.

BRIEF SUMMARY

The present invention relates to a catheter system according to claim 1. Further developments are given in the dependent claims.

Various embodiments are directed to an electrophysiological catheter system including a catheter shaft with proximal and distal ends, a catheter tip, a strain sensitive element, and wireless communication circuitry. The catheter tip is coupled to the distal end of the catheter shaft, and conducts diagnostics or therapies within a patient's vasculature. The strain sensitive element is coupled near a distal end of the catheter shaft, and deforms in response to a force translated from the catheter tip, through the elongated shaft, to the strain sensitive element. The deformation of the strain sensitive element fluctuates at least one electrical characteristic of the strain sensitive element, and the change in the electrical characteristic of the strain sensitive element is indicative of the force exerted on the catheter tip. The wireless communication circuitry is electrically coupled to the strain sensitive element, and wirelessly transmits a first electronic signal that is indicative of the force exerted on the catheter tip.

Other non-claimed embodiments for explaining the invention are directed to a non-invasive catheter device that includes a strain sensitive element and a resonant LC circuit. The strain sensitive element is coupled near a distal end of the catheter, and deforms in response to a force on the catheter. The deformation of the strain sensitive element is associated with the force exerted on the catheter. The resonant LC circuit includes an inductor and a capacitor coupled in series, and is electrically coupled to the strain sensitive element. The resonant LC circuit configured to receives a first wireless electrical signal near/or at a resonant frequency of the resonant LC circuit, and in response to the first wireless electrical signal an oscillatory voltage is induced that facilitates the transmission of a second wireless electrical signal that has an induced resonant frequency relative to the first wireless electrical signal, and based on the deformation of the strain sensitive element. Accordingly, the induced resonant frequency is indicative of the force exerted on the catheter.

The foregoing and other aspects, features, details, utilities, and advantages of the present disclosure will be apparent from reading the following description and claims, and from reviewing the accompanying drawings.

BRIEF DESCRIPTION OF THE DRAWINGS

Various example embodiments may be more completely understood in consideration of the following detailed description in connection with the accompanying drawings.

FIG. 1 is a schematic and diagrammatic view of a catheter system for performing a therapeutic medical procedure, consistent with various aspects of the present disclosure.
FIG. 2A is a cross-sectional front view of a distal portion of an ablation catheter, consistent with various aspects of the present disclosure.
FIG. 2B is a detailed cross-sectional front view of a wireless force sensor within the ablation catheter of FIG. 2A, consistent with various aspects of the present disclosure.
FIG. 3 is a side-view of a C-arm for fluoroscopic imaging encompassing a patient on an operating table, consistent with various aspects of the present disclosure.
FIG. 4 is a front view of a distal portion of a cardiac catheter including a printed wireless force sensor, consistent with various aspects of the present disclosure.
FIG. 5 is a catheter system including a front view of a distal portion of a cardiac catheter including a printed wireless force sensor and a communications subsystem, consistent with various aspects of the present disclosure.
FIGs. 6A-6C are front views of a length of a distal portion of a cardiac catheter including various capacitive wireless force sensor configurations, consistent with various aspects of the present disclosure.
FIGs. 7A-7C are front views of a length of a distal portion of a cardiac catheter including various resistive wireless force sensor configurations, consistent with various aspects of the present disclosure.
FIGs. 8A-8E are front views of a length of a distal portion of a cardiac catheter including various inductive wireless force sensor configurations, consistent with various aspects of the present disclosure.
FIG. 9A is a front view of a distal portion of a cardiac catheter including a resistive force sensor, consistent with various aspects of the present disclosure.
FIG. 9B is a front view of a distal portion of a cardiac catheter including a resistive force sensor, consistent with various aspects of the present disclosure.
FIG. 9C is a front view of a distal portion of a cardiac catheter including a resistive force sensor, consistent with various aspects of the present disclosure.
FIG. 10A is a front view of a distal portion of a cardiac catheter including a capacitive force sensor, consistent with various aspects of the present disclosure.
FIG. 10B is a front view of a distal portion of a cardiac catheter including a capacitive force sensor, consistent with various aspects of the present disclosure.
FIG. 10C is a front view of a distal portion of a cardiac catheter including a capacitive force sensor, consistent with various aspects of the present disclosure.

While various embodiments discussed herein are amenable to modifications and alternative forms, aspects thereof have been shown by way of example in the drawings and will be described in detail. It should be understood, however, that the intention is not to limit the invention to the particular embodiments described. On the contrary, the intention is to cover all modifications and alternatives falling within the scope of the disclosure including aspects defined in the claims. In addition, the term "example" as used throughout this application is only by way of illustration, and not limitation.

## DETAILED DESCRIPTION OF EMBODIMENTS

The instant disclosure for explaining the invention relates to wireless force sensors, in particular medical catheters with wireless force sensors embedded therein. In one embodiment, the instant disclosure relates to catheters for treating cardiac arrhythmias. In such embodiments, an embedded wireless force sensor on a distal surface of the catheter shaft may detect a force exerted on the tissue by the catheter and wirelessly transmit a signal indicative of the sensed force to a communications subsystem.

Various aspects of the present disclosure are directed to a low cost, easy to manufacture, wireless force sensor for catheter applications. Specific embodiments of the wireless force sensor utilize direct write electronic printing to form a wireless force sensor directly onto a catheter shaft. For example, the wireless force sensor may be printed onto the catheter shaft using direct-write electronic additive manufacturing techniques (e.g., aerosol jet, micropen, and ink jet applications of conductive traces and dielectrics). Due to the desirable characteristics of direct write materials, such as high conductivity inks, embodiments of the present disclosure are less cost/labor intensive for electrophysiological catheter applications. Such embodiments also facilitate smaller component sizes and assembly dimensional fits. High conductivity inks may be utilized throughout the various embodiments of the present disclosure, including components such as conductors and rings, as well as magnetic pick-up coils and wireless force sensors (as described in more detail herein).

In accordance with the present disclosure, force sensors, including printed wireless stress/strain type load cells (also referred to as strain sensitive elements) on a flexible shaft, are disclosed. These load cells may utilize a resonating RLC circuit to facilitate wireless signal transmission. Printed stress/strain type load cells fall into three categories: piezo-resistive, piezoelectric and capacitive. Currently, catheter applications using any of the three categories of load cells require electrical connections along the length of the catheter shaft to communicate a sensor signal. These electrical connections are prone to fatigue and failure during typical bending and deflection of a catheter during use, and require established electrode connections for proper functionality. In the event of a failure of the electrical connections, the sensors are rendered inoperable. Aspects of the present disclosure overcome such connectivity problems by utilizing wireless communication protocols between the force sensor and a communication subsystem. General teachings related to printed stress/strain load cells are disclosed in, for example, *Mattana*, "Recent Advances in Printed Sensors", Materials Today, 2015.

Various embodiments of the present disclosure are directed to piezo-resistive force sensors including those utilizing a Wheatstone bridge topography - such a topography may be highly sensitive to strain (e.g., strain detection of ~0.01%).

Details of the various embodiments of the present disclosure are described below with specific reference to the figures.

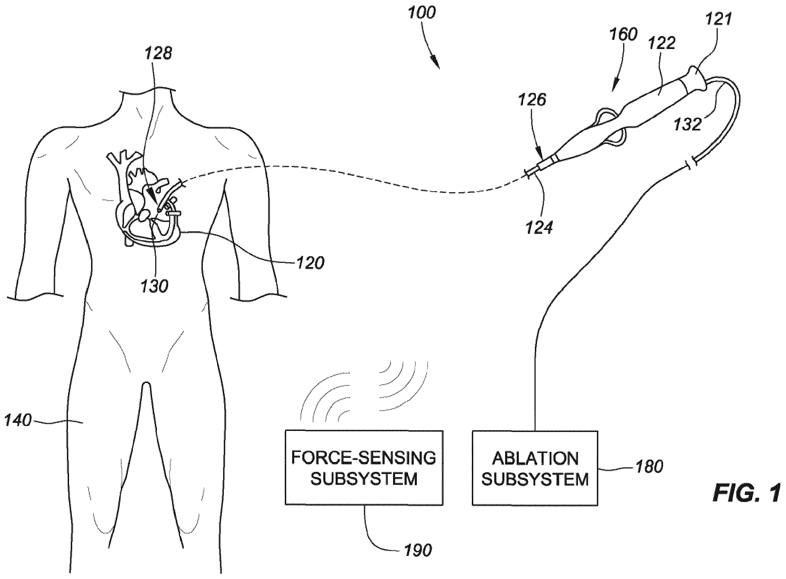

Referring now to the drawings wherein like reference numerals are used to identify similar components in the various views, Fig. 1 is a schematic and diagrammatic view of an electrophysiological catheter system 100 for performing diagnostics and therapies within a cardiac muscle of a human body 140, for example, ablation therapy of tissue 120. It should be understood, however, that catheter systems consistent with aspects of the present disclosure may find application in connection with a variety of other locations within human and non-human bodies, and therefore, the present disclosure is not meant to be limited to the use of the system in connection with only cardiac tissue and/or human bodies, or in regard to ablation therapies.

Electrophysiological catheter system 100 may include a catheter 160, an ablation subsystem 180 for controlling an ablation therapy conducted by an ablation catheter tip 130 at a distal end of the catheter. The ablation subsystem may control the application of and/or generation of ablative energy including, for example, radio frequency (RF), cryoablation, laser, chemical, and high-intensity focused ultrasound, among others. The catheter system may further include a force-sensing subsystem 190 (referred to hereinafter as a communication subsystem) for determining and indicating to a clinician when the ablation catheter tip comes in contact with myocardial tissue within the cardiac muscle (among other impediments), and optionally how much force is exerted upon the impediment by the ablation catheter tip. In various embodiments consistent with the present disclosure, the communication subsystem 190 wirelessly receives signals indicative of a force sensed and transmitted by a wireless force sensor on a distal portion of the catheter. In yet further embodiments, the communication subsystem 190 may transmit activation signals to the wireless force sensor that activate an RLC oscillator within the wireless force-sensor, and the communication subsystem 190 may be integrated within an ablation imaging/localization system (e.g., Mediguide™).

Ablation therapies often require precise force exertion for optimal ablative-energy transfer into the targeted myocardial tissue. Excess force between the ablation catheter tip and the targeted myocardial tissue may result in excessive ablation which may permanently damage the cardiac muscle and/or surrounding nerves. In contrast, contact force below a target force between the ablation catheter tip and the targeted myocardial tissue may reduce the efficacy of the ablation therapy, as insufficient ablative energy is transferred to the myocardial tissue. In such a case, myocardial tissue receiving the ablation therapy may regenerate and continue conducting electrical impulses. Accordingly, it is desirable for a clinician performing the ablation therapy to receive feedback from the distal tip of the catheter including the force exerted by the catheter on myocardial tissue being ablated.

In the exemplary embodiment of Fig. 1, catheter 160 is provided for examination, diagnosis, and/or treatment of internal body tissue such as cardiac tissue 120. The catheter may include a cable connector or interface 121, a handle 122, a shaft 124 having a proximal end 126 and a distal end 128 (as used herein, "proximal" refers to a direction toward the end of the catheter 160 near the handle 122, and "distal" refers to a direction away from the handle 122), and an ablation catheter tip 130 coupled to the distal end of the catheter shaft. In many embodiments, the handle may include inputs for a clinician to control the function of the catheter tip (e.g., ablation) and/or exert steering inputs for the catheter shaft.

As shown in Fig. 1, ablation catheter tip 130 may be manipulated through vasculature of a patient 140 using handle 122 to steer one or more portions of shaft 124 and position the ablation catheter tip at a desired location within heart 120. In various embodiments, the ablation catheter tip includes ablation elements (e.g., ablation electrodes, high intensity focused ultrasonic ablation elements, etc.) that when operated by ablation subsystem 180 ablates the tissue in contact with the ablation catheter tip (and in some cases tissue in proximity to the ablation catheter tip may be ablated by conductive energy transfer through the blood pool and to the proximal tissue). To verify that proper contact is maintained during an ablation therapy, a wireless force sensor near the ablation catheter tip 130 measures a force exerted on the tip by tissue in contact therewith and wirelessly transmits an electrical signal indicative of the sensed force. The wireless signal is received by the communication subsystem 190 which may conduct signal processing as well as analog-to-digital conversion of the signal before associating the electrical signal with an exerted force. In yet further embodiments, the communication subsystem 190 may transmit activation signals to the wireless force sensor that activate the RLC oscillator, and the communication subsystem 190 may be integrated within an ablation imaging/localization system. The communication subsystem may further display the calculated force for the clinician or otherwise intervene during the ablation therapy where necessary to maintain the efficacy of the therapy. For example, the communications subsystem 190 may communicate with ablation subsystem 180 to intervene in the therapy. In response to a sensed force being below a low-threshold, the ablation subsystem 180 may increase the duration of the therapy or the intensity, for example. Similarly, in response to a sensed force being above a high-threshold, the ablation subsystem 180 may decrease the duration or the intensity of the therapy, or abort the therapy.

In various specific embodiments of the present disclosure, catheter 160 may include electrodes and one or more positioning sensors (e.g., magnetic sensors) at a distal end 128 of catheter shaft 124. In such an embodiment, the electrodes may acquire electrophysiology data (also referred to as "EP data") relating to cardiac tissue within heart 120, while the positioning sensor(s) generates positioning data indicative of a 3-D position of the ablation catheter tip 130. In further embodiments, the catheter may include other conventional catheter components such as, for example and without limitation, steering wires and actuators, irrigation lumens and ports, contact sensors, temperature sensors, additional electrodes, and corresponding conductors or leads.

Connector 121 provides mechanical and electrical connection(s) for one or more cables 132 extending, for example, from ablation subsystem 180 to ablation catheter tip 130 mounted to a distal end 128 of catheter shaft 124. In other embodiments, the connector may also provide mechanical, electrical, and/or fluid connections for cables extending from other components in catheter system 100, such as, for example, a fluid source (when the catheter 160 includes an irrigated catheter tip). In reference to measuring a force exerted by the catheter tip, an RLC force sensor (by way of example) may require communication subsystem 190 to conduct back-end processing of a wireless output signal transmitted from the RLC sensor.

Handle 122 provides a location for a clinician to operate catheter 160 and may further provide steering or guidance inputs for catheter shaft 124 while inserted within a patient's body 140. For example, the handle may include means to manipulate one or more steering wires extending through the catheter to a distal end 128 of the shaft - thereby facilitating steering the shaft. The handle is conventional in the art and it will be understood that the construction of the handle may vary. In other embodiments, control of the catheter may be automated by robotically driving or controlling the catheter shaft, or driving and controlling the catheter shaft using a magnetic-based guidance system.

Catheter shaft 124 is an elongated, tubular, and flexible member configured for movement within a patient's body 140. The shaft supports an ablation catheter tip 130 at a distal end 128 of catheter 160. The shaft may also permit transport, delivery and/or removal of fluids (including irrigation fluids, cryogenic ablation fluids, and body fluids), medicines, and/or surgical tools or instruments. The shaft, which may be made from conventional materials used for catheters, such as polyurethane, defines one or more lumens configured to house and/or transport electrical conductors, fluids, surgical tools, and/or steering cables. The catheter may be introduced into a blood vessel or other structure within the body through a conventional introducer sheath.

In an exemplary cardiac ablation therapy, to correct for an atrial arrhythmia, the introducer sheath is introduced through a peripheral vein (typically a femoral vein) and advanced into a right atrium of a patient's cardiac muscle 120, in what is referred to as a transseptal approach. The introducer sheath then makes an incision in the fossa ovalis (the tissue wall between the left and right atriums), and extends through the incision in the fossa ovalis to anchor the introducer sheath in the fossa ovalis. The ablation catheter 160 may then be extended through a lumen of the introducer sheath into the left atrium. Catheter shaft 124 may then be steered or guided through the left atrium to position an ablation catheter tip 130 into a desired location within the left atrium such as in proximity to a pulmonary vein where an ablation therapy is to be applied. During application of an ablation therapy, a wireless force sensor printed along a length of a distal portion of the catheter shaft 124 may wirelessly transmit signals to communication subsystem 190 indicative of a force exerted on the ablation catheter 160 by the myocardial tissue. Accordingly, the clinician may adjust the force exerted to best suit a particular ablation therapy.

Aspects of the present disclosure improve the efficacy of ablation therapy by maintaining a consistent force between ablation catheter tip 130 and myocardial tissue being ablated during a single-point ablation, as well as along a lesion line comprising a number of individual ablations. In various embodiments, a wireless force sensor consistent with the present disclosure reduces the complexity of the catheter assembly, while reducing ambient noise in the resulting electrical signal indicative of a sensed force.

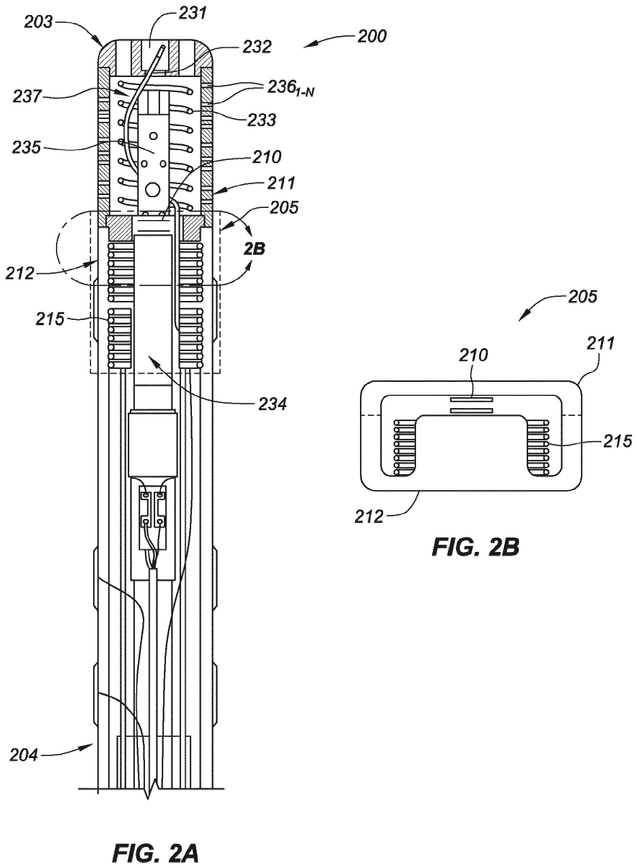

FIG. 2A is a cross-sectional side view of a distal portion 203 of an ablation catheter 200, consistent with various aspects of the present disclosure.

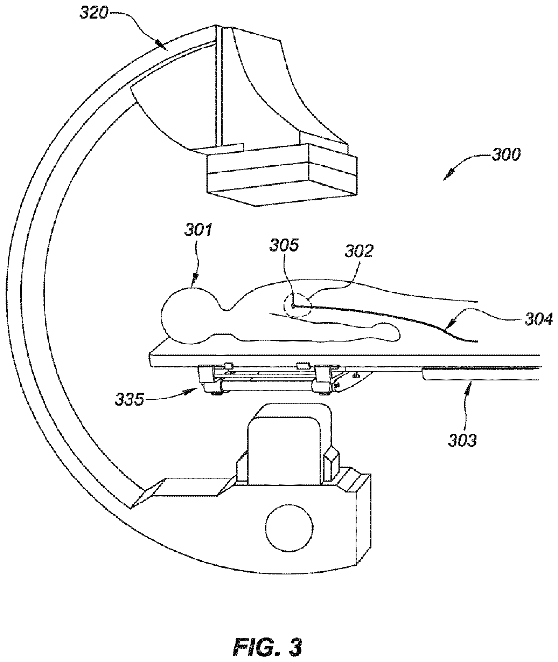

As shown in FIG. 2A, a wireless force sensor 205 is configured for use on a distal tip 203 of an ablation catheter 200. In yet other embodiments, the wireless force sensor 205 may be configured for use within an intravascular blood pressure (IVP) catheter. In yet further embodiments, the sensor 205 may be installed within the tip of an introducer sheath (such as an Agilis™ introducer sheath, produced by St. Jude Medical, Inc.) to eliminate the need for an IVP catheter entirely. The shape of inductive coil windings 215 of the sensor 205 (e.g., CardioMEMS sensor) are configured to fit within the distal tip 203 of the catheter 200, and the pressure or force sensitive capacitive element 210 is placed near the tip of the catheter. A communication subsystem (335, as shown in Fig. 3) may be placed under the patient. In some embodiments, the communication subsystem may be integrated into an operating table mattress, or placed underneath the operating table. In yet further more specific embodiments, the communication subsystem may be integrated into a table mount for a surgical visualization and navigation system, such as MediGuide™, a product manufactured and sold by St. Jude Medical, Inc..

Catheter 200 may include an RF electrode 231 at a distal tip 203 for ablating tissue in contact therewith. The RF electrode can be powered by a signal generator near a proximal end 204 of the catheter 200 that is communicatively coupled to the RF electrode by lead wire 232. During an ablation therapy, to prevent blood coagulation in proximity to the distal tip 203, an irrigant lumen 234 delivers irrigant fluid to a distal tip 203. The fluid is distributed throughout a chamber 237 within the distal tip 203 via manifold 235, and out apertures $236_{1-N}$ extending through deflecting portion 211. In various embodiments consistent with the present disclosure, the irrigant fluid may also facilitate cooling of the distal tip 203 during ablation therapy.

When distal tip 203 is placed into contact with tissue in preparation for conducting an ablation therapy, a force is exerted upon the tissue. In ablation therapy applications, the force exerted by the distal tip 203 of the catheter 200 is directly correlated with a therapy outcome. In more specific embodiments, a force above or below a threshold force may result in an undesirable therapy outcome - such as over-ablation, or failure to ablate the tissue, for example.

To better assist a clinician conducting an ablation therapy, embodiments of the present disclosure are directed toward a force sensor 205 for sensing a force exerted by, or on, a distal tip 203 of catheter 200. Specifically, a spring 233 within chamber 237 of the distal tip 203 may be compressed in response to a force exerted between the distal tip and tissue in contact therewith. A deflecting portion 211 of the distal tip 203 deflects in response to the force. A non-deflecting portion 212 and the force sensor 205 coupled thereto detects the deflection of the deflecting portion in response to the force exerted on the distal tip 203. As the deflection of the tip is associated with a force exerted on the tip, by way of the known spring constant of spring 233, the force exerted on the distal tip may be determined by the force sensor 205. In various embodiments, the force sensor consists of a wireless force sensor.

In one exemplary embodiment consistent with FIG. 2A, a force sensor 205 is configured for use within a non-invasive cardiac catheter device 200. The force sensor 205 may consist of a pressure or force sensitive capacitive element 210 which is electrically coupled to the inductive coil windings 215. The force sensor in the present embodiments forms a resonant LC circuit (such a combination of circuitry also referred to herein as a CardioMEMS sensor). The LC circuit resonance may be measured remotely using a communication subsystem; the communication between the LC resonance circuit and the communication subsystem may use one of a plurality of known wireless communication protocols. In one application, the capacitive element 210 may be used to measure intravascular blood pressure. In yet another application, the capacitive element may be used to measure catheter tip contact force in a cardiac ablation catheter.

FIG. 2B is a detailed view of a wireless force sensor 205 (e.g., CardioMEMS) within an ablation catheter 200 of FIG. 2A, and consistent with various aspects of the present disclosure. As shown in FIG. 2B, one half of capacitive element 210 is coupled to non-deflecting portion 212 and the other half is coupled to deflecting portion 211. In response to a force exerted on distal tip 203, spring 233 is compressed moving the deflecting portion 211 toward non-deflecting portion 212 (see, e.g., FIG. 2A) - thereby changing the distance between the two halves of capacitive element 210. The change in distance between the capacitive element portions affect the effective capacitance of capacitive element 210. In response to the change in capacitance of the capacitive element 210, the inductive coil windings 215 transmit a varying wireless signal, which is associated with a force exerted on the force sensor 205. Upon reception of the wireless signal by a communication subsystem (also referred to as a wireless transceiver), the received signal may be associated with a force exerted on distal tip 203 of catheter 200, and the force may be communicated to the clinician. For example, the force exerted by the catheter may be visually indicated on a dial or gauge, a varying audible tone, or tactile feedback - such as a vibration in the handle, among other well-known sensory communication techniques.

FIG. 3 is a side-view of an operating room 300 including a C-arm 320 for fluoroscopic imaging encompassing a patient 301 on a patient table 303 with a communication subsystem 335, consistent with various aspects of the present disclosure.

A catheter assembly 304 includes a catheter tip assembly 305 at a distal end portion. The catheter tip assembly may be operatively adapted for conducting a diagnostic or a therapeutic procedure under clinician control. A proximal end portion of the catheter 304 may include a steering handle or other mechanism (not shown). In the present embodiment, catheter 304 is an ablation therapy catheter. The catheter assembly 304 includes a flexible shaft extending between the proximal end portion and the catheter tip assembly 305. The catheter assembly may further include an electrical connector (not shown) configured to establish electrical connection(s) between the catheter tip assembly 305 and external electrical devices (not shown) to perform, for example, localization, mapping, ablation, and/or pacing procedures. The catheter tip assembly may comprise a plurality of localization sensor coils such as those shown in U.S. Patent No. 6,690,963 (see, e.g., sensors 30, 32, 34 depicted in FIGS. 2 and 3).

As shown in FIG. 3, a communication subsystem 335 receives signals from a wireless force-sensor at a catheter tip assembly 305, which is located within a cardiac muscle 302 of a patient 301, for example. In yet further embodiments, the communication subsystem 335 may transmit activation signals to the wireless force sensor that activate an RLC oscillator of the wireless force sensor, and the communication subsystem 335 may be integrated within an ablation imaging/localization system. In the present embodiment, the distal tip of the catheter 304 is positioned within the cardiac muscle 302 to conduct an ablation therapy. Ablation therapies are commonly used to necrose myocardial tissue in a cardiac muscle, which may alleviate symptoms associated with epicardial ventricular tachycardia, atrial fibrillation, and other cardiac conditions. One source of such electrical impulses is from arrhythmiatic foci located in the pulmonary veins. In atrial fibrillation, for example, myocardial tissue in a cardiac muscle forms a conductive pathway for electrical signals traveling through pulmonary venous tissue. The electrical signals from the pulmonary veins disrupt the electrical signals that trigger the orderly pumping motion of the cardiac muscle, resulting in an irregular, and often rapid heart rate that commonly causes poor blood flow. The ablation therapy electrically isolates the sources of such unwanted electrical impulses from the cardiac muscle by effecting necrosis of myocardial tissue between the pulmonary veins and the right atrium. By isolating these electrical impulses from the cardiac muscle via ablation therapy, symptoms relating to atrial fibrillation may be relieved.

In various embodiments of the present disclosure, communication subsystem 335 may be alternatively incorporated into a fluoroscopy imaging system, such as C-Arm 320; such a combination provides numerous benefits including, importantly, preventing the communication subsystem 335 from interfering with the quality of fluoroscopic imaging. This configuration also facilitates improved positioning of the communication subsystem 335 relative to the patient 301 without creating any further impediment between the clinician and the patient.

During an ablation therapy, a communication subsystem 335 activates a wireless force sensor and receives electrical signals transmitted from the sensor. The electrical signal is indicative of a force exerted by catheter 304 on myocardial tissue, for example. It has been discovered that transmitting electrical signals via wireless means, in catheter-related applications, reduces electrical noise-pickup and other electrical signal distortion and degradation associated with wired communication of such electrical signals along a length of the catheter 304 via lead wire(s), while also reducing the requisite diameter of the catheter shaft in some embodiments. Further, in catheter applications utilizing localization systems to determine a location of a distal tip of the catheter within a patient (e.g., magnetic localization, capacitive localization, and hybrid-type systems), lead wires extending along a length of the catheter shaft may act as a transformer, affecting the performance of both magnetic and capacitive localization systems.

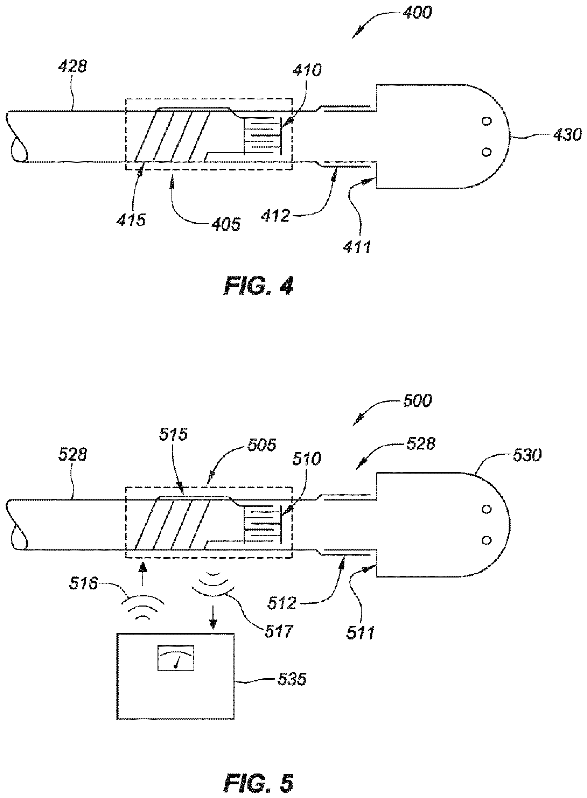

FIG. 4 is a front view of a distal portion 400 of a cardiac catheter including a printed wireless force sensor 405, consistent with various aspects of the present disclosure. Specifically, a strain sensitive resistive, capacitive, or inductive element 410 is printed on a flexible shaft segment 428 adjacent to the ablation tip 430. The strain sensitive element 410 is electrically coupled to induction coil 415 to form an RLC circuit that comprises wireless force sensor 405. Force applied to the ablation tip 430 generates a strain across the strain sensitive element 410 resulting in a change of capacitance, resistance, or inductance. This change in capacitance, resistance, or inductance is wirelessly sensed by activating the wireless force sensor 405 with an electromagnetic field, and measuring the emitted resonant frequency response of the activated force sensor 405. In various embodiments, a calibration relates the applied tip force to the resulting transmitted frequency response from the force sensor 405.

As shown in FIG. 4, a distal portion 400 of a cardiac catheter includes a deflecting portion 411. The deflecting portion 411 (relative to a non-deflecting portion 412) deflects in response to a force exerted on distal tip 430. As a result of the force, a strain is applied across the strain sensitive element thereby resulting in a change of capacitance, resistance, or inductance of the strain sensitive element 410. This change in capacitance, resistance, or inductance is wirelessly sensed by activating the wireless force sensing circuit 405 with an electromagnetic field, and measuring the emitted resonant frequency response of the activated circuit 405.

In general, a distal portion 400 of a catheter system, consistent with the present disclosure, may include a catheter tip 430, catheter shaft 428, a wireless RLC force sensor 405 including a printed piezo-resistor, capacitor, or inductor 410 and printed induction coil 415. In other embodiments, including active wireless circuitry, the above embodiment may further include electronic circuitry for both receiving activation signals and transmitting strain dependent emission signals.

In one particular application, an activation signal transmitted by a communications subsystem 535 (as shown in Fig. 5) may be in proximity to/ or at the resonant frequency of the RLC circuit 505 to induce an oscillatory voltage. The frequency, bandwidth, and attenuation of the subsequent induced resonant frequency may depend on the values of one or more of the capacitance, resistance, or inductance of strain sensitive element 510. For example, a decrease in capacitance will result in an increase in the LC resonant frequency. Alternately, a change in resistance is expected to result in a change of fractional bandwidth or quality factor - resulting in an output signal of the RLC circuit 505 associated with an applied tip force.

FIG. 5 is a catheter system 500 including a front view of a distal portion 528 of a cardiac catheter including a printed wireless force sensor 505 and a communication subsystem 535 (e.g., a wireless transceiver), consistent with various aspects of the present disclosure. During a catheter-based procedure, a clinician may activate the wireless transceiver 535 which transmits an activation signal 516 - such as an electromagnetic field to induce resonance to the printed wireless force sensor 505. The electromagnetic field when received by induction coil 515 generates an electrical current that powers the printed wireless force sensor 505 including a printed piezo-resistor, capacitor, or inductor elements 510 that strains in response to a force on the catheter tip. Printed induction coil 515 both receives the electromagnetic field and transmits a response based on the sensed strain. In non-passive embodiments of wireless force sensor 505, the sensor may also include transmission elements that facilitate the transmission of a strain dependent emission signal 517 back to the wireless transceiver 535. In such an embodiment, the wireless transceiver 535 may associate the strain dependent emission signal 517 with a calibrated, or otherwise associated, force exerted upon the distal tip 530.

A distal portion 500 of a cardiac catheter, as shown in FIG. 5, includes a deflecting portion 511. The deflecting portion 511 deflects (relative to a non-deflecting portion 512) in response to a force exerted on distal tip 530. As a result of the force, a strain is applied across the strain sensitive element 510 which facilitates the flexing of portions of the printed strain sensitive element 510 relative to itself - thereby resulting in a change of capacitance, resistance, or inductance of the strain sensitive element 510. This change in capacitance, resistance, or inductance induces a resonant frequency response in the resulting strain dependent emission signal 517. When the wireless transceiver 535 receives the signal 517 from the printed wireless force sensor, circuitry within the wireless transceiver 535 associates the signal 517 with a force exerted on the distal tip 530 of the catheter. In various embodiments, the association between the signal 517 and the force exerted on the distal tip 530 of the catheter may be based on, for example, a look-up table, formula, or other calibration means.

In some embodiments, a wireless transceiver transmission signal 516 powers the wireless force sensor 505 and a difference between signals 516 and 517 are indicative of a sensed force.

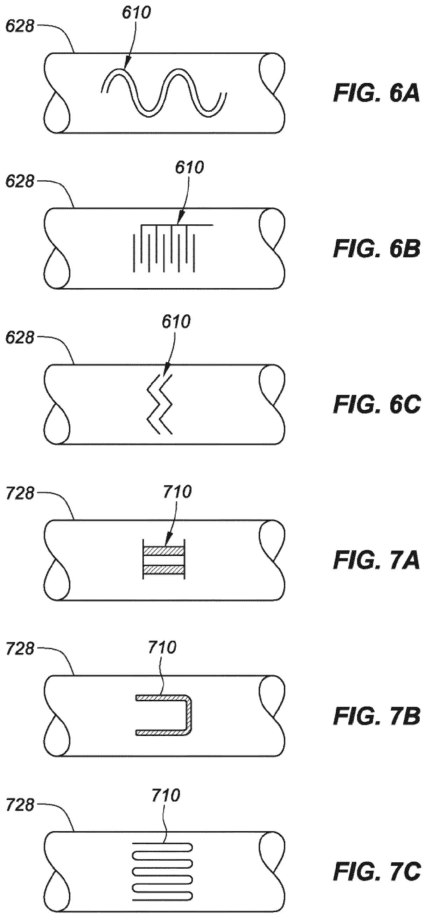

FIGs. 6A-6C are front views of a length 628 of a distal portion of a catheter including a capacitive strain sensitive element 610 of a wireless force sensor, consistent with various aspects of the present disclosure. In FIGs. 6A-6C, the capacitive strain sensitive elements 610 are a capacitor printed on an exterior portion of the length 628 of the catheter shaft. In many embodiments consistent with FIGs. 6A-6C, the capacitor may have both conducting and dielectric regions. For a parallel plate capacitor (see e.g., FIGs. 6A-6C), the relationship between a change in capacitance ($\Delta C$) and elongation of the capacitive strain sensitive element from applied tip load ($\Delta d$) is given by:

$$\frac{\Delta C}{\Delta d} = -\varepsilon \frac{A}{d^2}$$

Eq. 1

where $\varepsilon$ is the medium dielectric constant and A is the electrode surface area. Accordingly, in a first approximation the output capacitance change may be increased by decreasing the distance between electrodes, increasing surface area, and increasing the medium dielectric constant; whereas the signal-to-noise is predominately increased by decreasing the electrode spacing.

A dielectric medium of a capacitive strain sensitive element 610 may include any material which is not electrically conducting. In specific embodiments, a dielectric medium of the capacitive strain sensitive element 610 may include materials such as polymers and ceramics. In various embodiments consistent with the present disclosure, due to capacitance versus strain signal output increasing with a dielectric constant (also referred to as permittivity) of the medium, it may be desirable that the dielectric medium be composed of very high permittivity materials. Examples of such materials may include solid polymer electrolytes (e.g., polyethylene glycol ("PEG") based polymers having dissolved lithium salts), ionomeric materials (e.g., Nafion perfluoropolyether ion exchange resins), and flexible polymers having large permanent dipole moments (e.g., polyvinylidene fluoride ("PVDF")), among others. Furthermore, it may be desirable that the dielectric medium be of high compliance to maximize incorporation of the imposed strain field from the applied force.

In view of Equation 1, the capacitance of the strain sensitive element 610 will be proportional to a surface area (A) of the printed conductor. As the strain sensitive element 610 is substantially electrically conducting, a large variety of conducting inks may be used to print the strain sensitive element on a length 628 of a distal portion of the catheter shaft. Embodiments of the present disclosure may utilize conducting inks including, for example, metallic flake filled epoxies, nanoparticle silver, gold and copper based inks, among others. In yet further more specific embodiments, the conducting inks used in the strain sensitive element may be based on highly conjugated carbon (e.g., nanoparticle carbon nanotubes), conducting polymers such as polythiophene, and the like.

In addition to increasing capacitor surface area of a strain sensitive element 610 via microstructures of conductive printed ink, the capacitive electrodes may be formed in a geometry which also optimizes capacitance. These optimized capacitive geometries may include, for example, zig-zag patterns or interdigitated fingers as illustrated in FIGs. 6A-6C - all of which maximize capacitor surface area for a given foot-print on a shaft 628 of the catheter.

FIGs. 7A-7C are front views of a length 728 of a distal portion of a catheter including a resistive strain sensitive element 710 of a wireless force sensor, consistent with various aspects of the present disclosure. In such embodiments, the resistive strain sensitive element 710 is a piezo-resistor. In various wireless force sensor configurations consistent with the present disclosure, the RLC wireless transmitter including the resistive strain sensitive element, and may also require an additional capacitor. In specific applications, the capacitance of the RLC wireless transmitter capacitor may correspond to the inherent stray capacitance within the conducting ink of the resistive strain sensitive element 710. For a resistive strain sensitive element, the relation between relative resistance change (ΔR/R) and strain (ΔL/L) is given by:

$$\frac{\Delta R}{R} = G \cdot \frac{\Delta L}{L}$$

Eq. 2

where G is the gauge factor that is dependent on both the overall dimensions of the resistive strain sensitive element and the strain dependence of bulk resistivity.

The strain resistive composition of a resistive strain sensitive element 710 may be any material which is electrically conducting and deforms under an applied load. For printed conductors, such strain resistive compositions may include micro-structured materials. It may be particularly desirable in some specific embodiments that the strain resistive composition include micro-structured materials including a conducting phase that percolates thru a matrix via a network of connected domains. Similar to the conducting regions of capacitive strain sensitive elements (as discussed in reference to FIGs. 6A-C), the materials used to form the resistive strain sensitive element may be any of the typical inks used in current electronic printing techniques. Specifically, the electronic printing inks for the resistive strain sensitive elements may include conducting carbon inks such as carbon nanotubes, graphene, or a combination thereof. The resistive strain sensitive element 710 may also include conducting metallic inks such as flakes, and nanoparticle dispersions of silver, gold, and copper, among others. Nanoparticle platinum based inks are particularly preferred for resistive strain sensitive elements. Furthermore, the strain sensitivity of the resistive composition may be optimized during the ink sintering process.

Various manufacturing processes may be utilized to achieve a desired application of electronic printing inks on a surface of a catheter shaft 728. For example, the ink may be sintered for a temperature and length of time such that interconnected conducting particles of the electronic inks are at a percolation threshold, thereby enhancing strain sensitivity of the resulting resistive formulation.

The geometry of a resistive formulation for a resistive strain sensitive element 710 may also be optimized for strain sensitivity. In general, the resistive strain sensitive element 710 may have a geometry with a high aspect ratio, wherein a longitudinal axis is parallel to the predicted direction of strain. It has been discovered that a high aspect ratio improves a signal-to-noise ratio of a sensed strain.

In FIG. 7A, parallel dual resistive elements comprise resistive strain sensitive element 710. The parallel, dual resistive elements have a decreased length due in part to the width of each resistive element which allows for an increased surface area with minimal length. Moreover, the resistive strain sensitive element 710 of the FIG. 7A embodiment has a high-aspect ratio, with a longitudinal axis parallel to the predicted direction of strain - facilitating improved signal strength of a strain placed along the longitudinal axis of catheter shaft 728.

In FIG. 7B, resistive strain sensitive element 710 forms a horse-shoe shape with a majority of the length of the element being parallel to the predicted direction of strain - thereby facilitating improved resolution of the strain sensed by the strain sensitive element 710.

FIG. 7C shows a strain sensitive element 710 configured in a zig-zag type pattern, with a majority of the length of the element parallel to the predicted direction of strain (along a longitudinal axis of the catheter shaft 728). The length and zig-zag pattern of the element facilitating a narrower width, while maintaining a large overall surface area of the strain sensitive element.

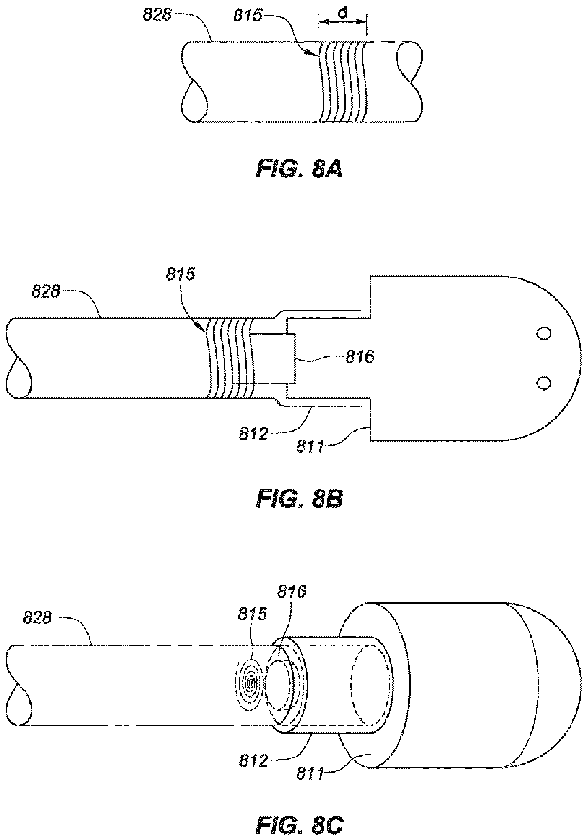

FIGs. 8A-8E are front views of a length 828 of a distal portion of a cardiac catheter including various inductive wireless force sensors, consistent with various aspects of the present disclosure. An inductance change in a printed RLC circuit including the wireless force sensor can be used to sense applied force on a shaft 828. Examples of various force sensing inductive strain sensitive elements 815 are shown in FIGs. 8A-8E. As shown in FIG. 8A, the inductive strain sensitive element 815 is a circumferentially extending coil that is printed on a flexible portion of shaft 828. As the distance (d) of the total inductor coil changes due to the loading on a distal tip, the inductance will change according to the relationship expressed by the following equation:

$$L = u_0 u_r n^2 A/d$$

$$\text{Eq. 3}$$

Where $u_0$ is the magnetic permeability of free space, $u_r$ is the relative magnetic permeability of magnetic core material, n is the number of turns, A is the cross-sectional area of the inductor, and d is the length of the inductor.

In the embodiment shown in FIGs. 8B and 8C, a ferromagnetic material 816 with high magnetic permeability is used to tune the inductance of the inductive strain sensitive element 815. As a force is applied to the distal tip, the ferromagnetic material 816 moves closer to the inductive strain sensitive element 815 making the inductance higher - as the ferromagnetic material 816 is located on a deformable portion 811 and the inductive strain sensitive element 815 is coupled to a static portion 812. Both the inductive strain sensitive element 815 and the ferromagnetic material 816 can be printed on to an exterior or interior portion of shaft 828. In order to simplify the fabrication of an RLC circuit, including the strain or force sensitive element comprising the inductive strain sensitive element 815, turn-to-turn parasitic capacitance can be used as a capacitance, realizing a complete RLC circuit with only solenoid or spiral coils, thereby mitigating the need for a separate resonating capacitance connection.

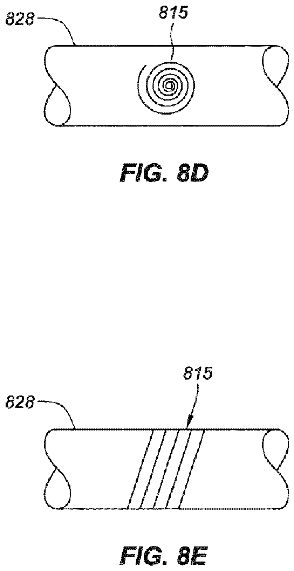

An inductive strain sensitive element 815 may also be formed from conducting inks. Alternative embodiments of the induction coil are shown in FIGs. 8D and 8E. In general the RLC circuit including the inductive strain sensitive element 815 may include geometry which is capable of acting as an antenna to both activate the circuit thru a received, applied electromagnetic field, and transmit the subsequent resonant frequency resulting from the induced voltage/current. In one specific embodiment, a reading wire loop may be placed inside the catheter shaft 828 to improve sensitivity.

In FIG. 8D, the inductive strain sensitive element 815 is a spiraled coil printed on a outer/inner diameter of catheter shaft 828. Alternatively, the spiraled coil may be positioned within the catheter shaft along a plane extending substantially parallel with a longitudinal axis of the catheter shaft.

In FIG. 8E, inductive strain sensitive element 815 is printed/wrapped circumferentially about an inner/outer surface of catheter shaft 828.

A communication subsystem may include both a transmitter for activating an electromagnetic field, and a receiver to capture the subsequent resonant signal transmitted by the PLC circuit in response. Outputs which may be related to applied force/strain include peak frequency, bandwidth, and characteristic dissipation times, for example. The communication subsystem may also utilize a S11 measurement method, CardioMEMS method (see, e.g., U.S. Patent No. 7,245,117) or a more basic communication method (e.g., ping and listen).

In various specific embodiments, flexible shaft 828 may be attached to the ablation tip such that a significant amount of the tip applied load is transferred to the flexible shaft, which is comprised of an insulating polymer.

In some specific implementations, more than one strain sensitive element 815 can be printed around the circumference of the flexible shaft 828. Such an implementation may be advantageous for determining the directionality of the applied force. Furthermore, multiple strain sensitive elements may be used for improved signal stability. For example, orthogonally positioned capacitive or resistive strain sensitive elements may be used to compensate for temperature variation. In such multiple strain sensing element embodiments, both axial and non-axial forces exerted on the distal tip of the catheter may be sensed.

A wireless force sensor consistent with the present disclosure is intended to include both powered and un-powered (e.g., active and passive, respectively) circuitry. For example, in some embodiments, wireless force sensor circuitry is passive and merely modulates a received frequency from a communications subsystem. In other embodiments, the wireless force sensor circuitry may be an active system with a power source (e.g., capacitor) that facilitates regular wake-ups of an integrated circuit to measure a strain on a strain sensitive element 815, and to transmit wireless signals to the communications subsystem indicative of the strain. In such an embodiment, the wireless force sensor circuitry may receive a high-frequency signal from the communications subsystem that facilitates charging of the power source to maintain operation of the wireless force sensor circuitry. In active systems, the deformation of the strain sensitive element may amplify the resulting signal transmission. In yet more specific embodiments, the wireless force sensor circuitry at a distal end of a catheter shaft may be electrically coupled with a power source at a proximal end of the catheter shaft to provide continuous power to the force sensor circuitry, while mitigating the need for power supply storage and driver circuitry at the distal tip of the catheter shaft. In such an embodiment, electrical signals indicative of the strain on the strain sensitive element may still be wirelessly communicated with the communication subsystem - as the wireless communication of such a signal mitigates noise pick-up along a length of the catheter shaft, and reduces catheter shaft size and complexity.

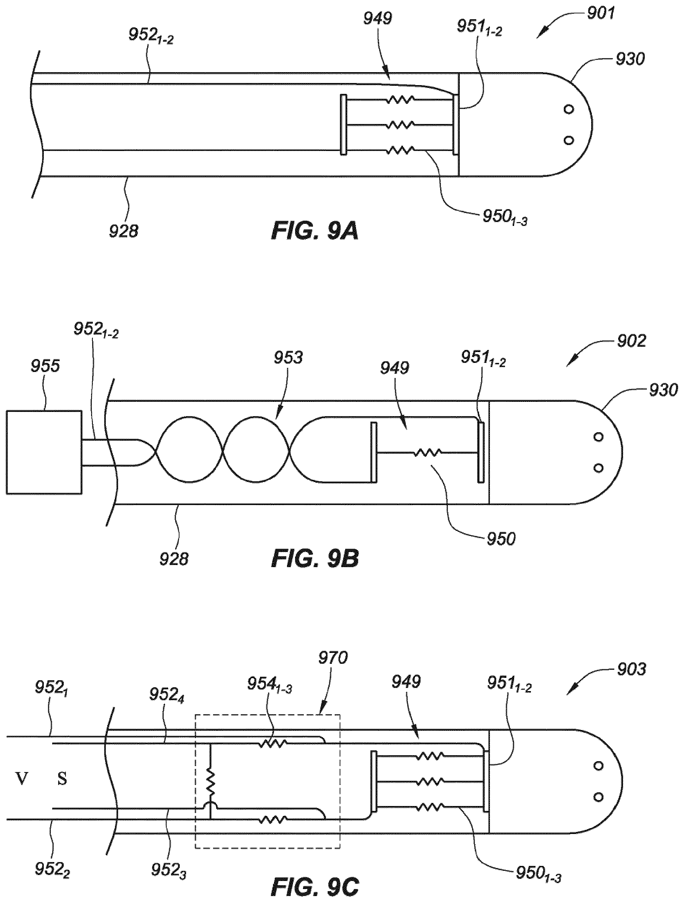

FIG. 9A is a side view of a distal portion 901 of a cardiac catheter including a force sensor 949. One or more strain sensitive elements $950_{1-3}$ are printed on a flexible shaft 928 attached adjacent to an ablation tip 930. Force applied to the ablation tip 930 generates a strain within the flexible shaft 928 resulting in a change of resistance within one or more of the strain sensitive elements $950_{1-3}$. To sense the change in resistance across the one or more of the strain sensitive elements $950_{1-3}$, a voltage may be applied across contacts $951_{1-2}$ (via conductors/wires $952_{1-2}$). Controller circuitry communicatively coupled to the conductors/wires $952_{1-2}$ may apply the voltage across the strain sensitive elements $950_{1-3}$ and measure a change in current flow in response to a strain exerted on the flexible shaft 928 via the ablation tip 930. In some embodiments the conductors/wires $952_{1-2}$ and/or contacts $951_{1-2}$ may also be printed on the flexible shaft 928 or routed through a lumen of the catheter shaft 928.

The one or more strain sensitive elements $950_{1-3}$ may be circumferentially distributed about the catheter shaft 928 to facilitate measuring strain exerted upon distal tip 930 in at least three degrees of freedom.

Depending on the material characteristics of catheter shaft 928, a substrate may be coated over the catheter shaft 928 to facilitate additive printing.

Various components of a force sensor 949 may be printed on to a flexible shaft 928 via electronic additive manufacturing; for example, aerosol jet, micropen, inkjet and contact printing application of conductive traces and dielectrics. The use of such additive, high-conductivity inks facilitate reduced labor/cost for the catheter assemblies as disclosed herein.

FIG. 9B is a side view of a distal portion 902 of a cardiac catheter including a force sensor 949. The force sensor includes a strain sensitive element 950 printed on a flexible shaft 928 attached adjacent to an ablation tip 930. Force applied to the ablation tip 930 generates a strain within the flexible shaft 928 resulting in a change of resistance within the strain sensitive element 950. To sense the change in resistance across the strain sensitive element 950, a voltage may be applied across contacts $951_{1-2}$ (via conductors/wires $952_{1-2}$). Controller circuitry 955 communicatively coupled to the conductors/wires $952_{1-2}$ may apply the voltage across the strain sensitive element 950, and measure a change in current flow in response to a strain exerted on the flexible shaft 928 via the ablation tip 930. In some embodiments the conductors/wires $952_{1-2}$ and/or contacts $951_{1-2}$ may also be printed on the flexible shaft 928 or routed through a lumen of the catheter shaft 928. Furthermore, some embodiments may utilize a twisted-pair configuration 953 of the conductors to mitigate electrical interference (noise) induced from external electromagnetic fields on a strain signal communicated between the strain sensitive element 950 and the controller circuitry 955. In some embodiments, the controller circuitry may be located within a catheter handle of the cardiac catheter, or communicatively coupled thereto. To further mitigate electrical interference, analog-to-digital circuitry may be positioned within the cardiac catheter as close to the strain sensitive element 950 as possible. The digital signal output of the analog-to-digital circuitry is less prone to electrical interference than the analog signal.

Controller circuitry 955 may provide an electronic signal dependent on the resistance of the strain sensitive element 950. The input and output signals of the controller circuitry may be direct current and/or alternating current.

The contacts $951_{1-2}$ may be any conductive material to provide electrical connection to the strain sensitive element 950. The contacts may generally comprise materials with higher electrical conductivity and reduced strain dependence (compared to the strain sensitive element 950). In some embodiments, the contacts may be formed of similar material to the strain sensitive element, but with enhanced conductivity such as by sintering at higher temperatures or a longer period of time. Material for the contacts may be selected from a set of conducting inks such as carbon, silver, gold, and copper for example. The conductors/wires $952_{1-2}$ may also be formed from similar conducting inks. In some embodiments, the conductors/wires $952_{1-2}$ may comprise stranded wires subsequently bonded/soldered to the contacts $951_{1-2}$.

Flexible shaft 928 is coupled to ablation tip 930 in such a way as to facilitate efficient transfer of a tip-applied load to the shaft. In some embodiments, the flexible shaft may comprise an insulating polymer or a composition of insulating polymers.

The embodiment of FIG. 9B may be limited in its ability to sense strain exerted axially relative to a longitudinal axis of catheter shaft 928, and compression/tension exerted on the ablation tip 930 in a single plane that is trans-axial with the longitudinal axis of the catheter shaft.

In specific embodiments, strain sensitive element 950 may be communicatively coupled to controller circuitry 955 including a Wheatstone bridge topography, where the sensing signal is the voltage difference between the two branches of the Wheatstone bridge.

FIG. 9C is a side view of a distal portion 903 of a cardiac catheter including a force sensor 949, consistent with various aspects of the present disclosure. As shown in FIG. 9C, a Wheatstone bridge sensing element 970 (including resistors $954_{1-3}$ electrically coupled between the force sensor 949, input voltage V (between conductors $952_{1-2}$), and output signal voltage S (between conductors $952_{3-4}$)), may be entirely printed on a distal portion 903 of catheter shaft 928. The Wheatstone bridge sensing element 970 is communicatively coupled to strain sensative element 949. An input voltage, V, is applied across contacts $951_{1-2}$ and initially balanced Wheatstone bridge sensing element 970. An output signal voltage, S, results from force sensor 949, including one or more strain sensitive elements $950_{1-3}$, being induced to strain along catheter shaft 928. Aspects of the present disclosure may exhibit improved signal stability due at least in part to isolation of sensing signal, S, from changes in conductor resistance along the catheter shaft.

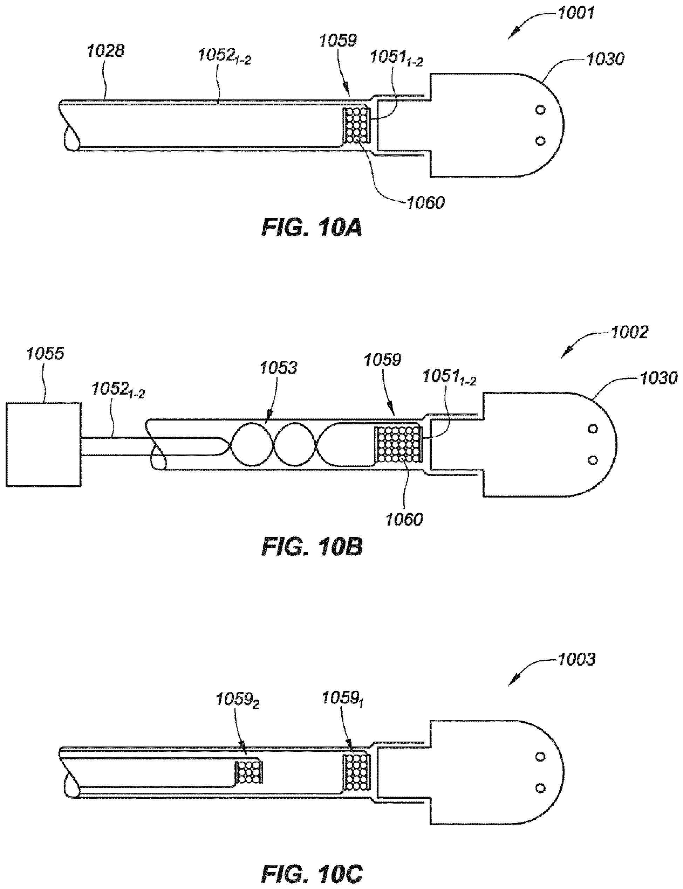

FIG. 10A is a side view of a distal portion 1001 of a cardiac catheter including a force sensor with one or more strain sensitive elements 1059, consistent with various aspects of the present disclosure. The one or more strain sensitive elements 1059 may be capacitive strain sensitive elements. One or more of the strain sensitive elements 1059 may be printed on a flexible shaft 1028 attached adjacent to an ablation tip 1030. In Fig. 10A, the strain sensitive element 1059 includes two contacts/electrodes $1051_{1-2}$ which are separated by a dielectric layer 1060. Material for the contacts may be selected from a set of conducting inks such as carbon, silver, gold, and copper for example. Conductors/wires $1052_{1-2}$ communicatively coupled to the contacts may also be formed from similar conducting inks.

Force applied to the ablation tip 1030 generates a strain within the flexible shaft 1028 resulting in a change of capacitance within one or more of the capacitive strain sensitive elements 1059. To sense the change in capacitance across the one or more of the capacitive strain sensitive elements 1059, a current is driven across contacts/electrodes $1051_{1-2}$ (via conductors/wires $1052_{1-2}$). As the characteristics of the electrodes are known, and the change in voltage across the capacitive strain sensitive element 1059 may be readily measured, a change in distance between the electrodes associated with a strain on the catheter shaft 1028 may be calculated.

In some embodiments, conductors/wires $1052_{1-2}$ and/or contacts $1051_{1-2}$ may also be printed on the flexible shaft 1028, or routed through a lumen of the catheter shaft.

The one or more strain sensitive elements $1050_{1-3}$ may be circumferentially distributed about the catheter shaft 1028 to facilitate measuring strain exerted upon distal tip 1030 in at least three degrees of freedom.

Depending on the material characteristics of catheter shaft 928, a substrate may be coated over the catheter shaft 1028 to facilitate the additive printing.

The various components of a force sensor may be printed on to a flexible shaft 1028 via electronic additive manufacturing; for example, aerosol jet, micropen, ink-jet and contact printing application of conductive traces and dielectrics. The use of such additive, high-conductivity inks facilitate reduce labor/cost for such catheter assemblies as disclosed herein.

FIG. 10B is a side view of a distal portion 1002 of a cardiac catheter including a force sensor with one or more strain sensitive elements 1059, consistent with various aspects of the present disclosure. Aspects of the present embodiment are further directed to the one or more strain sensitive elements 1059 being capacitive strain sensitive elements. One or more of the strain sensitive elements 1059 are printed on a flexible shaft 1028 attached adjacent to an ablation tip 1030. The strain sensitive element 1059 includes two contacts/electrodes $1051_{1-2}$ which are separated by a dielectric layer 1060.

Force applied to the ablation tip 1030 generates a strain within the flexible shaft 1028 resulting in a change of capacitance within one or more of the strain sensitive elements 1059. To sense the change in capacitance across the one or more of the strain sensitive elements 1059, a current is driven across contacts/electrodes $1051_{1-2}$ (via conductors/wires $1052_{1-2}$). As the characteristics of the electrodes is known, and the change in voltage across the strain sensitive element 1059 may be readily measured, a change in distance between the electrodes associated with a strain on the catheter shaft 1028 may be determined. Controller circuitry 1055 coupled to the conductors/wires $1052_{1-2}$ may be tasked with determining the strain across the strain sensitive element 1059 by measuring the capacitance of the strain sensitive element in response to a strain exerted on the ablation tip 1030.

In some embodiments the conductors/wires $1052_{1-2}$ and/or contacts $1051_{1-2}$ may also be printed on the flexible shaft 1028 or routed through a lumen of the catheter shaft. Furthermore, some embodiments may utilize a twisted-pair configuration 1053 of the conductors to mitigate electrical interference (noise) induced from external electromagnetic fields on a strain signal communicated between the strain sensitive element 1059 and the controller circuitry 1055. In some embodiments, the controller circuitry may be located within a catheter handle of the cardiac catheter, or communicatively coupled thereto. To further mitigate electrical interference, analog-to-digital circuitry may be positioned within the cardiac catheter as close to the strain sensitive element 1059 as possible. The digital signal output of the analog-to-digital circuitry being less prone to electrical interference then the analog signal.

Controller circuitry 1055 may provide an electronic signal dependent on the resistance of the strain sensitive element. The input and output signals of the controller circuitry may be direct current and/or alternating current. In one specific embodiment, the controller circuitry may produce a periodic alternating current input for strain sensitive element 1059, and monitor an associated output. Any parameter of the output which is affected by the capacitance of the strain sensitive element 1059 may be used to determine the applied tip load. Calibration to associate a range of strain sensitive element 1059 capacitances to applied loads at the ablation tip 1030 may be desirable for enhanced accuracy. Output parameters may include, for example, magnitude of complex impedance, phase angle, resistance or reactance, etc.

More than one force sensor may be printed circumferentially about flexible shaft 1028. Furthermore, the distal electrode 1051 may be comprised of the ablation tip 1030.

FIG. 10C is a side view of a distal portion 1003 of a cardiac catheter including a force sensor with strain sensitive element $1059_1$(which in the present embodiment is a strain sensitive capacitor), consistent with various aspects of the present disclosure. In the present embodiment, a reference capacitor $1059_2$ is located proximal relative to a strain sensitive capacitor $1059_1$. The reference capacitor $1059_2$ is not subjected to tip load induced strain, only the strain sensitive element $1059_1$. To improve the calibration and increase signal-to-noise ratio of an output signal from the strain sensitive element $1059_1$, a comparative output signal is emitted from the reference capacitor $1059_2$ which is merely indicative of the noise (as the reference capacitor is not subject to catheter shaft strain). Controller circuitry, communicatively coupled to the strain sensitive element $1059_1$ and reference capacitor $1059_2$ may receive the output signals from both and filter out the noise from the strain sensitive element's signal. In some specific embodiments, the controller circuitry may sense a phase angle difference between the outputs of the strain sensitive element $1059_1$ and reference capacitor $1059_2$. The phase angle difference is associated with an ambient electrical noise in proximity to the ablation tip 1030, and facilitates the (partial) filtering of the ambient electrical noise from the strain sensitive element $1059_1$ output signal.

In various embodiments of the present disclosure, strain sensitive element $1059_1$ and reference capacitor $1059_2$ may have varying structural characteristics. For example, the reference capacitor $1059_2$ may be tuned to receive frequencies commonly associated with ambient electrical noise.

While the embodiments of FIGs. 9A-10C are illustrated as wired force sensor configurations, these embodiments may be readily implemented as wireless force sensors (as fully enabled herein).

Although several embodiments have been described above with a certain degree of particularity, those skilled in the art could make numerous alterations to the disclosed embodiments without departing from the scope of the present disclosure. It is intended that all matter contained in the above description or shown in the accompanying drawings shall be interpreted as illustrative only and not limiting. Changes in detail or structure may be made without departing from the present teachings. The foregoing description and following claims are intended to cover all such modifications and variations.

Various embodiments are described herein of various apparatuses, systems, and methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are nonlimiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," "an embodiment," or the like, means that a particular feature, structure, or characteristic described in connection with the embodiment is included in at least one embodiment. Thus, appearances of the phrases "in various embodiments," "in some embodiments," "in one embodiment," "in an embodiment," or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features structures, or characteristics of one or more other embodiments without limitation.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

**Claims**

1. An electrophysiological catheter system comprising:

   a catheter shaft (428, 528, 628, 728, 828, 928, 1028) including proximal and distal ends;
   a catheter tip (430, 530, 930, 1030) coupled to the distal end of the catheter shaft (428, 528, 628, 728, 828, 928, 1028);
   a strain sensitive element (410, 510, 610, 710, 815, 950, 1059) coupled on the distal end of the catheter shaft (428, 528, 628, 728, 828, 928, 1028), the strain sensitive element (410, 510, 610, 710, 815, 950, 1059) configured and arranged to deform in response to a force translated from the catheter tip (430, 530, 930, 1030), through the catheter shaft (428, 528, 628, 728, 828, 928, 1028), to the strain sensitive element (410, 510, 610, 710, 815, 950, 1059), the deformation of the strain sensitive element (410, 510, 610, 710, 815, 950, 1059) fluctuating at least one electrical characteristic of the strain sensitive element (410, 510, 610, 710, 815, 950, 1059), and the change in the at least one electrical characteristic of the strain sensitive element (410, 510, 610, 710, 815, 950, 1059) indicative of the force exerted on the catheter tip (430, 530, 930, 1030); and
   wireless communication circuitry electrically coupled to the strain sensitive element (410, 510, 610, 710, 815, 950, 1059), the wireless communication circuitry configured and arranged to wirelessly transmit a first electronic signal that is indicative of the force exerted on the catheter tip (430, 530, 930, 1030), **characterized in that**
   the wireless communication circuitry is further configured and arranged to receive a second electrical signal, store the energy received from the second electrical signal, monitor a change in at least one of the electrical characteristics of the strain sensitive element over time, and periodically transmit the first electrical signal, wherein
   the wireless communication circuitry includes an induction coil (415, 515) that is electrically coupled to the strain sensitive element (410, 510, 610, 710, 815, 950, 1059), the induction coil (415, 515) being configured and arranged to receive the second electrical signal, wherein the second electrical signal is at or near a resonant frequency of the induction coil (415, 515) which induces an oscillatory voltage in the strain sensitive element (410, 510, 610, 710, 815, 950, 1059) and which modulates the second electrical signal to form the first electrical signal in response to deformation of the strain sensitive element (410, 510, 610, 710, 815, 950, 1059).

2. The catheter system of claim 1, wherein the first electronic signal is modulated by the fluctuation of the at least one electrical characteristic of the strain sensitive element (410, 510, 610, 710, 815, 950, 1059) as the strain sensitive element (410, 510, 610, 710, 815, 950, 1059) is deformed in response to the force exerted on the catheter tip (430, 530, 930, 1030).

3. The catheter system of claim 1, further including a power source at a proximal end of the catheter shaft (428, 528, 628, 728, 828, 928, 1028), the power source electrically coupled to the wireless communication circuitry via lead wires extending from the proximal end to the distal end of the catheter shaft (428, 528, 628, 728, 828, 928, 1028).

4. The catheter system of claim 1, wherein the at least one electrical characteristic of the strain sensitive element (410, 510, 610, 710, 815, 950, 1059) includes one or more of the following: resistance, inductance, and capacitance.

5. The catheter system of claim 1, wherein the wireless communication circuitry includes an induction coil (415, 515) that is electrically coupled to the strain sensitive element (410, 510, 610, 710, 815, 950, 1059), and the induction coil (415, 515) is configured and arranged to modulate a transmission frequency of the first electrical signal in response to the fluctuation of the at least one electrical characteristic of the strain sensitive element (410, 510, 610, 710, 815, 950, 1059).

6. The catheter system of claim 1, wherein the strain sensitive element (410, 510, 610, 710, 815, 950, 1059) includes a Wheatstone bridge.

7. The catheter system of claim 1, wherein the strain sensitive element (410, 510, 610, 710, 815, 950, 1059) is further configured and arranged to measure an intravascular blood pressure.

8. The catheter system of claim 1, wherein the strain sensitive element (410, 510, 610, 710, 815, 950, 1059) is further configured and arranged, in response to the deformation force, to fluctuate at least one of capacitance, inductance, and resistance characteristics, thereby modifying the first electrical signal prior to being wirelessly transmitted by the wireless communication circuitry.

9. The catheter system of claim 1, wherein the strain sensitive element (410, 510, 610, 710, 815, 950, 1059) and the wireless communication circuitry are coupled to the catheter shaft (428, 528, 628, 728, 828, 928, 1028) using direct-write electronic additive manufacturing.

**Patentansprüche**

1. Elektrophysiologisches Kathetersystem, mit:

   einem Katheterschaft (428, 528, 628, 728, 828, 928, 1028), das ein proximales und ein distales Ende aufweist;
   einer Katheterspitze (430, 530, 930, 1030), die mit dem distalen Ende des Katheterschafts (428, 528, 628, 728, 828, 928, 1028) gekoppelt ist;
   einem dehnungsempfindlichen Element (410, 510, 610, 710, 815, 950, 1059), das mit dem distalen Ende des Katheterschafts (428, 528, 628, 728, 828, 928, 1028) gekoppelt ist, wobei das dehnungsempfindliche Element (410, 510, 610, 710, 815, 950, 1059) konfiguriert und angeordnet ist zur Verformung in Antwort auf eine Kraft, die von der Katheterspitze (430, 530, 930, 1030) über den Katheterschaft (428, 528, 628, 728, 828, 928, 1028) an das dehnungsempfindliche Element (410, 510, 610, 710, 815, 950, 1059) übertragen wird, wobei die Verformung des dehnungsempfindlichen Elements (410, 510, 610, 710, 815, 950, 1059) mindestens eine elektrische Eigenschaft des dehnungsempfindlichen Elements (410, 510, 610, 710, 815, 950, 1059) ändert, und die Änderung in der mindestens einen elektrischen Eigenschaft des dehnungsempfindlichen Elements (410, 510, 610, 710, 815, 950, 1059) kennzeichnend ist für die Kraft, die auf die Katheterspitze (430, 530, 930, 1030) wirkt; und
   einer drahtlosen Kommunikationsschaltung, die mit dem dehnungsempfindlichen Element (410, 510, 610, 710, 815, 950, 1059) elektrisch gekoppelt ist, wobei die drahtlose Kommunikationsschaltung konfiguriert und angeordnet ist zur drahtlosen Übertragung eines ersten elektronischen Signals, das kennzeichnend ist für die Kraft, die auf die Katheterspitze (430, 530, 930, 1030) wirkt, **dadurch gekennzeichnet, dass** die drahtlose Kommunikationsschaltung ferner konfiguriert und angeordnet ist zum Empfangen eines zweiten elektrischen Signals, zum Speichern der von dem zweiten elektrischen Signal empfangenen Energie, zum Überwachen einer zeitlichen Änderung in mindestens einer von den elektrischen Eigenschaften des dehnungsempfindlichen Elements, und zum periodisches Übertragen des ersten elektrischen Signals, wobei die drahtlose Kommunikationsschaltung eine Induktionsspule (415, 515) aufweist, die mit dem dehnungsempfindlichen Element (410, 510, 610, 710, 815, 950, 1059) elektrisch gekoppelt ist, wobei die Induktionsspule (415, 515) konfiguriert und angeordnet ist zum Empfangen des zweiten elektrischen Signals, wobei das zweite elektrische Signal bei oder nahe bei einer Resonanzfrequenz der Induktionsspule (415, 515) ist, die eine Oszillationsspannung in dem dehnungsempfindlichen Element (410, 510, 610, 710, 815, 950, 1059) induziert, und die das zweite elektrische Signal moduliert zum Bilden des ersten elektrischen Signals in Antwort auf die Verformung des dehnungsempfindlichen Elements (410, 510, 610, 710, 815, 950, 1059).

2. Kathetersystem nach Anspruch 1, bei dem das erste elektronische Signal durch die Änderung der mindestens einen elektrischen Eigenschaft des dehnungsempfindlichen Elements (410, 510, 610, 710, 815, 950, 1059) moduliert wird, wenn sich das dehnungsempfindliche Element (410, 510, 610, 710, 815, 950, 1059) in Antwort auf die Kraft, die auf die Katheterspitze (430, 530, 930, 1030) wirkt, verformt.

3. Kathetersystem nach Anspruch 1, ferner mit einer Leistungsquelle an einem proximalen Ende des Katheterschafts (428, 528, 628, 728, 828, 928, 1028), wobei die Leistungsquelle über Leitungsdrähte, die sich von dem proximalen Ende zu dem distalen Ende des Katheterschafts (428, 528, 628, 728, 828, 928, 1028) erstrecken, mit der drahtlosen Kommunikationsschaltung elektrisch gekoppelt ist.

4. Kathetersystem nach Anspruch 1, bei dem die mindestens eine elektrische Eigenschaft des dehnungsempfindlichen Elements (410, 510, 610, 710, 815, 950, 1059) eines oder mehreres aufweist von: Widerstand, Induktivität und Kapazität.

5. Kathetersystem nach Anspruch 1, bei dem die drahtlose Kommunikationsschaltung eine Induktionsspule (415, 515) aufweist, die mit dem dehnungsempfindlichen Element (410, 510, 610, 710, 815, 950, 1059) elektrisch gekoppelt ist, und die Induktionsspule (415, 515) konfiguriert und angeordnet ist zur Modulation einer Übertragungsfrequenz des ersten elektrischen Signals in Antwort auf die Änderung der mindestens einen elektrischen Eigenschaft des dehnungsempfindlichen Elements (410, 510, 610, 710, 815, 950, 1059).

6. Kathetersystem nach Anspruch 1, bei dem das dehnungsempfindliche Element (410, 510, 610, 710, 815, 950, 1059) eine Wheatstone-Brücke aufweist.

7. Kathetersystem nach Anspruch 1, bei dem das dehnungsempfindliche Element (410, 510, 610, 710, 815, 950, 1059) ferner konfiguriert und angeordnet ist zum Messen eines intravaskulären Blutdrucks.

8. Kathetersystem nach Anspruch 1, bei dem das dehnungsempfindliche Element (410, 510, 610, 710, 815, 950, 1059) ferner konfiguriert und angeordnet ist, um in Antwort auf die Verformungskraft mindestens eines von Kapazitäts-, Induktivität- und Widerstandscharakteristik zu ändern, wodurch das erste Signal modifiziert wird, bevor es drahtlos von dem drahtlose Kommunikationsschaltung übertragen wird.

9. Kathetersystem nach Anspruch 1, bei dem das dehnungsempfindliche Element (410, 510, 610, 710, 815, 950, 1059) und die drahtlose Kommunikationsschaltung mit dem Katheterschaft (428, 528, 628, 728, 828, 928, 1028) gekoppelt sind, indem eine Direct-Write (DW) elektronisch additive Fertigung verwendet wird.

**Revendications**

1. Système de cathéter électrophysiologique comprenant :

   une tige de cathéter (428, 528, 628, 728, 828, 928, 1028) comprenant des extrémités proximale et distale ;
   une pointe de cathéter (430, 530, 930, 1030) couplée à l'extrémité distale de la tige de cathéter (428, 528, 628, 728, 828, 928, 1028) ;
   un élément sensible à la contrainte (410, 510, 610, 710, 815, 950, 1059) couplé à l'extrémité distale de la tige de cathéter (428, 528, 628, 728, 828, 928, 1028), l'élément sensible à la contrainte (410, 510, 610, 710, 815, 950, 1059) configuré et agencé pour se déformer en réponse à une force transmise de la pointe de cathéter (430, 530, 930, 1030), par l'intermédiaire de la tige de cathéter (428, 528, 628, 728, 828, 928, 1028), à l'élément sensible à la contrainte (410, 510, 610, 710, 815, 950, 1059), la déformation de l'élément sensible à la contrainte (410, 510, 610, 710, 815, 950, 1059) faisant varier au moins une caractéristique électrique de l'élément sensible à la contrainte (410, 510, 610, 710, 815, 950, 1059), et la variation de ladite au moins une caractéristique électrique de l'élément sensible à la contrainte (410, 510, 610, 710, 815, 950, 1059) indiquant la force exercée sur la pointe de cathéter (430, 530, 930, 1030) ; et
   un circuit de communication sans fil couplés électriquement à l'élément sensible à la contrainte (410, 510, 610, 710, 815, 950, 1059), le circuit de communication sans fil étant configuré et agencé pour transmettre sans fil un premier signal électronique qui est indicatif de la force exercée sur la pointe de cathéter (430, 530, 930, 1030), **caractérisé en ce que** le circuit de communication sans fil est en outre configuré et agencé pour recevoir un deuxième signal électrique, stocker l'énergie reçue du deuxième signal électrique, surveiller un changement d'au moins une des caractéristiques électriques de l'élément sensible à la contrainte au fil du temps, et transmettre de manière périodique le premier signal électrique, dans lequel le circuit de communication sans fil comprend une bobine d'induction (415, 515) qui est couplée électriquement à l'élément sensible à la contrainte (410, 510, 610, 710, 815, 950, 1059), la bobine d'induction (415, 515) étant configurée et agencée pour recevoir le second signal électrique, dans lequel le second signal électrique est à ou proche d'une fréquence de résonance de la bobine d'induction (415, 515) qui induit une tension oscillatoire dans l'élément sensible à la contrainte (410, 510, 610, 710, 815, 950, 1059) et qui module le second signal électrique pour former le premier signal électrique en réponse à la déformation de l'élément sensible à la contrainte (410, 510, 610, 710, 815, 950, 1059).

2. Système de cathéter selon la revendication 1, dans lequel le premier signal électronique est modulé par la fluctuation de ladite au moins une caractéristique électrique de l'élément sensible à la contrainte (410, 510, 610, 710, 815, 950, 1059) lorsque l'élément sensible à la contrainte (410, 510, 610, 710, 815, 950, 1059) est déformé en réponse à la force exercée sur la pointe de cathéter (430, 530, 930, 1030).

3. Système de cathéter selon la revendication 1, comprenant en outre une source d'alimentation à une extrémité proximale de la tige de cathéter (428, 528, 628, 728, 828, 928, 1028), la source d'alimentation étant couplée électriquement au circuit de communication sans fil par l'intermédiaire de fils conducteurs s'étendant de l'extrémité proximale à l'extrémité distale de la tige de cathéter (428, 528, 628, 728, 828, 928, 1028).

4. Système de cathéter selon la revendication 1, dans lequel ladite au moins une caractéristique électrique de l'élément sensible à la contrainte (410, 510, 610, 710, 815, 950, 1059) comprend une ou plusieurs des caractéristiques suivantes : résistance, inductance et capacité.

5. Système de cathéter selon la revendication 1, dans lequel le circuit de communication sans fil comprennent une bobine d'induction (415, 515) qui est couplée électriquement à l'élément sensible à la contrainte (410, 510, 610, 710, 815, 950, 1059), et la bobine d'induction (415, 515) est configurée et agencée pour moduler une fréquence de transmission du premier signal électrique en réponse à la fluctuation de ladite au moins une caractéristique électrique de l'élément sensible à la contrainte (410, 510, 610, 710, 815, 950, 1059).

6. Système de cathéter selon la revendication 1, dans lequel l'élément sensible à la contrainte (410, 510, 610, 710, 815, 950, 1059) comprend un pont de Wheatstone.

7. Système de cathéter selon la revendication 1, dans lequel l'élément sensible à la contrainte (410, 510, 610, 710, 815, 950, 1059) est en outre configuré et agencé pour mesurer une pression sanguine intravasculaire.

8. Système de cathéter selon la revendication 1, dans lequel l'élément sensible à la contrainte (410, 510, 610, 710, 815, 950, 1059) est en outre configuré et agencé, en réponse à la force de déformation, pour faire fluctuer au moins une des caractéristiques de capacité, d'inductance et de résistance, modifiant ainsi le premier signal électrique avant d'être transmis sans fil par le circuit de communication sans fil.

9. Système de cathéter selon la revendication 1, dans lequel l'élément sensible à la contrainte (410, 510, 610, 710, 815, 950, 1059) et le circuit de communication sans fil sont couplés au tige de cathéter (428, 528, 628, 728, 828, 928, 1028) en utilisant une fabrication additive électronique à écriture directe.

FIG. 1

FIG. 2A

FIG. 2B

**FIG. 3**

**FIG. 4**

**FIG. 5**

628

*610*

**FIG. 6A**

628

*610*

**FIG. 6B**

628

*610*

**FIG. 6C**

728

*710*

**FIG. 7A**

728

*710*

**FIG. 7B**

728

*710*

**FIG. 7C**

**FIG. 8A**

**FIG. 8B**

**FIG. 8C**

828 815

**FIG. 8D**

815

828

**FIG. 8E**

$952_{1\text{-}2}$     949     $951_{1\text{-}2}$     901

930

928     **FIG. 9A**     $950_{1\text{-}3}$

$952_{1\text{-}2}$     953     949     $951_{1\text{-}2}$     902

955     930

928     **FIG. 9B**     950

$952_1$    $952_4$    $954_{1\text{-}3}$   970    949    $951_{1\text{-}2}$    903

V   S

$952_2$    $952_3$    **FIG. 9C**    $950_{1\text{-}3}$

25

FIG. 10A

FIG. 10B

FIG. 10C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007050960 A2
- US 20140276078 A1
- EP 2347726 A2
- US 9101734 B2
- EP 2338430 A1
- WO 2017070322 A1
- US 6690963 B
- US 7245117 B